# EUROPÄISCHE PATENTSCHRIFT

(11) **EP 4 257 042 B1**
(45) Veröffentlichungstag und Bekanntmachung des Hinweises auf die Patenterteilung: **08.07.2026**
(21) Anmeldenummer: 23166429.3
(22) Anmeldetag: 04.04.2023
(51) Int. Cl.: A61B 5/107, A61B 17/17, A61B 17/00, A61B 5/00, A61B 90/00, A61F 2/46, A61F 2/38

(54) **MEHRZWECKMESSINSTRUMENT ZUR VERWENDUNG BEI EINER KNIEGELENKERSATZOPERATION**
MULTIPURPOSE MEASURING INSTRUMENT FOR USE IN KNEE REPLACEMENT SURGERY
INSTRUMENT DE MESURE À USAGES MULTIPLES DESTINÉ À ÊTRE UTILISÉ DANS UNE OPÉRATION DE REMPLACEMENT D'ARTICULATION DU GENOU

(30) Priorität: 06.04.2022 DE 102022203411
(43) Veröffentlichungstag der Anmeldung: 11.10.2023
(73) Patentinhaber: Aesculap AG, 78532 Tuttlingen (DE)
(72) Erfinder: Firmbach, Franz-Peter, 78576 Emmingen-Liptingen (DE); Anhorn, Svenja, 72535 Heroldstatt (DE)
(74) Vertreter: Patentanwälte Ruff, Wilhelm, Beier, Dauster & Partner mbB

(56) Entgegenhaltungen:
- CN-U- 211 094 130
- US-A1- 2016 030 053
- US-A1- 2016 278 873

## Beschreibung

Die Erfindung betrifft ein Mehrzweckmessinstrument zur Verwendung bei einer Kniegelenkersatzoperation, aufweisend einen Stab, welcher entlang einer Längsachse längserstreckt ist und einends eine entlang einer Querachse abragende erste Backe aufweist, wobei die erste Backe eine parallel zu der Querachse erstreckte erste Innenfläche aufweist, welche zur Anlage an einer Vorderseite einer Knochenresektion eingerichtet ist, einen Schieber, welcher entlang der Längsachse linearbeweglich an dem Stab geführt ist und einends eine entlang der Querachse abragende zweite Backe aufweist, wobei die zweite Backe eine parallel zu der Querachse erstreckte zweite Innenfläche aufweist, welche entgegengesetzt zu der ersten Innenfläche orientiert und zur Anlage an einer Rückseite der Knochenresektion eingerichtet ist, und aufweisend eine Skale, welche zwischen dem Stab und dem Schieber ausgebildet und wenigstens zur Anzeige einer entlang der Längsachse erstreckten Dicke der Knochenresektion eingerichtet ist.

Die Verwendung von orthopädischen Prothesen als künstlicher Ersatz für beschädigte oder abgenutzte natürliche Knochenstrukturen eines Patienten ist gängige medizinische Praxis. Insbesondere Hüft- und Kniegelenkersatzoperationen gehören mittlerweile zum Standardrepertoire der chirurgischen Orthopädie.

Bei einer Kniegelenkersatzoperation (englisch: Total Knee Arthroplasty (TKA)) werden abgenutzte oder anderweitig durch Krankheit oder einen Unfall beeinträchtigte Gelenkflächen des Femurs und/oder der Tibia durch eine Kniegelenkprothese ersetzt. Solche Kniegelenkprothesen umfassen üblicherweise eine Femurkomponente, welche am distalen Ende des Femurs implantiert wird, und eine Tibiakomponente, welche am proximalen Ende der Tibia implantiert wird. Um eine einwandfreie Funktion des künstlichen Gelenkersatzes zu gewährleisten, müssen die besagten Komponenten hinsichtlich ihrer Lage und Orientierung in Bezug auf die Anatomie des Patienten und dessen Körperachsen in definierter Weise möglichst präzise positioniert werden. Andernfalls ist mit einem für den Patienten nicht zufriedenstellenden Ergebnis zu rechnen. Hinsichtlich der Positionierung der Komponenten existieren unterschiedliche chirurgische Ansätze.

Ein als Mechanical Alignment bekannter und bislang üblicher Ansatz sieht vor, dass die Position und Ausrichtung der künstlichen Gelenkachsen der Kniegelenkprothese mechanisch ideal und insoweit ohne Berücksichtigung etwaiger orthopädischer Fehlstellungen des Patienten ausgerichtet werden. Hierbei dient oftmals die Längsachse der Tibia als Referenzachse für die Ausrichtung und Positionierung. Klinische Studien haben gezeigt, dass der Mechanical Alignment-Ansatz zu einer als unnatürlich empfundenen Funktion des künstlichen Kniegelenks führen kann.

Als ein weiterer Ansatz ist das sog. Kinematic Alignment bekannt. Bei dieser Vorgehensweise werden die Femurkomponente und die Tibiakomponente unter Berücksichtigung etwaiger orthopädischer Fehlstellungen des Patienten positioniert. Ziel hierbei ist es, die natürliche und unter Umständen mit Fehlstellungen behaftete Gelenkausrichtung des Patienten wiederherzustellen. Klinische Studien haben gezeigt, dass der Kinematic Alignment-Ansatz oftmals mit einer verbesserten Patientenzufriedenheit einhergeht. Insbesondere die Funktion des künstlichen Kniegelenks wird seitens der Patienten als eher natürlich empfunden.

Mit dem Bestreben, die Patientenzufriedenheit weiter zu verbessern, geht ein grundsätzlicher Bedarf nach möglichst präzisen, einfach verwendbaren und kosteneffizienten chirurgischen Instrumenten zur Umsetzung des Kinematic Alignment einher.

Die vorliegende Erfindung befasst sich mit solchen chirurgischen Instrumenten, genauer mit einem Mehrzweckmessinstrument zur Verwendung bei einer Kinematic Alignment TKA.

Ein derartiges Mehrzweckmessinstrument ist aus der US 10,582,982 B2 bekannt und zur Verwendung bei einer Kniegelenkersatzoperation nach dem Kinematic Alignment-Ansatz vorgesehen. Das bekannte Mehrzweckmessinstrument weist einen längserstreckten Stab und einen entlang der Längsachse des Stabs linearbeweglich geführten Schieber auf. Der Stab weist einends eine entlang einer Querachse abragende erste Backe mit einer ersten Innenfläche auf. Die erste Innenfläche ist zur Anlage an einer Vorderseite einer femoralen Knochenresektion eingerichtet. Der Schieber weist hierzu entsprechend eine zweite Backe mit einer zweiten Innenfläche auf, welche entgegengesetzt zu der ersten Innenfläche orientiert und zur Anlage an einer Rückseite der femoralen Knochenresektion eingerichtet ist. Weiter weist das bekannte Mehrzweckmessinstrument eine erste Skale auf. Diese ist der ersten Innenfläche und der zweiten Innenfläche zugeordnet und zur Anzeige einer entlang der Längsachse erstreckten Dicke der zwischen den Backen befindlichen femoralen Knochenresektion eingerichtet. Das bekannte Mehrzweckmessinstrument erlaubt zudem die Messung des sog. AP-Offsets zwischen dem distalen Femur der proximalen Tibia. Der AP-Offset bezeichnet den Abstand zwischen distalem Femur und proximaler Tibia entlang der Anterior-Posterior-Achse, wobei die Messung unter Flexion erfolgt. Zu diesem Zweck weist das bekannte Mehrzweckmessinstrument eine an dem Stab angeordnete dritte Backe auf, die entgegengesetzt zu der ersten Backe entlang der Querachse einends von dem Stab abragt. Zur Messung des AP-Offsets wird eine Stirnfläche der dritten Backe an die proximale Tibia angelegt und eine der zweiten Innenfläche abgewandte zweite Außenfläche der zweiten Backe wird an den distalen Femur angelegt. Zur Anzeige des AP-Offsets ist eine zweite Skale vorhanden. Diese ist auf einer der ersten Skale abgewandten Rückseite des Mehrzweckmessinstruments angeordnet. Im Übrigen ist das bekannte Mehrzweckmessinstrument als Wegwerfartikel gestaltet und zur lediglich einmaligen Verwendung vorgesehen.

Das in der US10,582,982B2 beschriebene Mehrzweckinstrument wird ebenfalls in der EP23166429 offenbart.

Aufgabe der Erfindung ist es, ein Mehrzweckmessinstrument der eingangs genannten Art bereitzustellen, das einen vereinfachten Aufbau aufweist und kosteneffizient herstellbar sowie einsetzbar ist.

Diese Aufgabe wird dadurch gelöst, dass ein Verlängerungsteil lösbar mit der ersten Backe verbunden ist, wobei das Verlängerungsteil eine zur Anlage an einer proximalen Tibia eingerichtete dritte Innenfläche aufweist, die parallel zu der Querachse erstreckt ist und weiter von dem Stab abragt als die erste Innenfläche, und dass die zweite Backe eine parallel zu der Querachse erstreckte zweite Außenfläche aufweist, welche entgegengesetzt zu der zweiten Innenfläche orientiert und zur Anlage an einem distalen Femur eingerichtet ist, wobei die dritte Innenfläche und die erste Innenfläche zum einen und zum anderen die zweite Innenfläche und die zweite Außenfläche jeweils entlang der Längsachse um einen identischen Abstand voneinander beabstandet sind, wodurch die Skale zusätzlich zur Anzeige eines Längsabstands zwischen dem distalen Femur und der proximalen Tibia eingerichtet ist. Durch die erfindungsgemäße Lösung kann insbesondere auf eine weitere Skale zur Anzeige des Längsabstands zwischen der proximalen Tibia und dem distalen Femur, d.h. dem AP-Offset, verzichtet werden. Dies wird durch die erfindungsgemäß paarweise identische Beabstandung der dritten und der ersten Innenfläche einerseits und der zweiten Innenfläche und der zweiten Außenfläche andererseits erreicht. Hierdurch ist die Skale gleichermaßen zum Anzeigen der Dicke der Knochenresektion und des AP-Offsets eingerichtet und/oder geeignet. Im Vergleich zu aus dem Stand der Technik bekannten Lösungen mit zwei gesonderten Skalen ergeben sich zum einen Kostenvorteile bei der Herstellung des Mehrzweckmessinstruments. Zudem ergibt sich eine vereinfachte Handhabung des Mehrzweckmessinstruments. Da lediglich eine (einzige) Skale vorhanden ist, werden Ablesefehler vermieden. Solche Ablesefehler sind bei aus dem Stand der Technik bekannten Lösungen grundsätzlich denkbar, indem beispielsweise der AP-Offset an der für die Dickenmessung eingerichteten Skale abgelesen wird oder umgekehrt. Eine solche Fehlzuordnung ist bei der erfindungsgemäßen Lösung ausgeschlossen. Zudem erlaubt das erfindungsgemäß vorhandene Verlängerungsteil einen besonders flexiblen und damit kosteneffizienten Einsatz des Mehrzweckmessinstruments. Das Verlängerungsteil ist zu diesem Zweck lösbar mit der ersten Backe verbunden. Hierdurch ist das erfindungsgemäße Mehrzweckmessinstrument auf einfache Weise zwischen einer ersten Konfiguration und einer zweiten Konfiguration überführbar. In der ersten Konfiguration ist das Verlängerungsteil lösbar mit der ersten Backe verbunden. In der zweiten Konfiguration ist das Verlängerungsteil von der ersten Backe gelöst. Die erste Konfiguration dient einer Messung des AP-Offsets. Zu diesem Zweck ragt die dritte Innenfläche des Verlängerungsteils entlang der Querachse weiter von dem Stab ab als die erste Innenfläche. Vereinfacht ausgedrückt fungiert das Verlängerungsteil als eine Art Verlängerung der ersten Backe. Hierdurch kann die dritte Innenfläche - unter gleichzeitiger Anlage der zweiten Außenfläche an dem distalen Femur - zur Messung des AP-Offsets an der proximalen Tibia angelegt werden. Hierzu im Unterschied ragt die erste Innenfläche hierfür nicht weit genug von dem Stab ab. Eine Messung des AP-Offsets über eine entsprechende Anlage der ersten Innenfläche und der zweiten Außenfläche ist folglich nicht möglich. In der zweiten Konfiguration ist das Verlängerungsteil von der ersten Backe gelöst. Diese Konfiguration des Mehrzweckmessinstruments dient der Messung der Dicke der Knochenresektion. Vorzugsweise überragt die dritte Innenfläche die zweite Innenfläche derart, dass eine Dickenmessung zwischen den besagten Innenflächen nicht möglich ist. Hierdurch werden Fehlmessungen infolge einer nicht bestimmungsgemäßen Verwendung des Verlängerungsteils zur Dickenmessung vermieden. Die lösbare Verbindung mit der ersten Backe gewährleistet ein einfaches Abnehmen und Anbringen des Verlängerungsteils. In unterschiedlichen Ausgestaltungen ist das Verlängerungsteil auf unterschiedliche Weise lösbar mit der ersten Backe verbunden, beispielsweise mittels einer Klemm-, Rast- oder Steckverbindung.

In Ausgestaltung der Erfindung ist das Verlängerungsteil mittels einer werkzeuglos lösbaren und werkzeuglos verbindbaren ersten Fügeverbindung mit der ersten Backe verbunden. Werkzeuglos lösbar meint, dass zum Lösen der ersten Fügeverbindung keinerlei Werkzeug notwendig ist. Dementsprechend meint werkzeuglos verbindbar, dass zum Verbinden der ersten Fügeverbindung keinerlei Werkzeug notwendig ist. Folglich kann das Verlängerungsteil bei dieser Ausgestaltung der Erfindung ohne Werkzeug an der ersten Backe angebracht und/oder von derselben entfernt werden. Hierdurch werden der Aufbau und die Handhabung des Mehrzweckmessinstruments weiter vereinfacht. Insbesondere kann eine Zeiteinsparung beim Wechsel zwischen den besagten unterschiedlichen Konfigurationen des Mehrzweckmessinstruments erreicht werden.

In weiterer Ausgestaltung der Erfindung weist die erste Fügeverbindung wenigstens eine erste Bohrung und ein erstes Bolzenelement auf, welche orthogonal zur Längs- und zur Querachse lösbar miteinander zusammengesteckt sind, wobei die erste Bohrung in die erste Backe eingebracht und das erste Bolzenelement fest mit dem Verlängerungsteil verbunden ist oder umgekehrt. Diese Ausgestaltung ist besonders einfach herstellbar und erlaubt eine robuste und zuverlässige lösbare Verbindung des Verlängerungsteils mit der ersten Backe. Bei dieser Ausgestaltung ist die erste Fügeverbindung eine zwischen der ersten Bohrung und dem ersten Bolzenelement ausgebildete lösbare Steckverbindung. Die Steckverbindung ist zwischen einem Innenumfang der ersten Bohrung und einem Außenumfang des ersten Bolzenelements ausgebildet. Dabei ist der Außenumfang vorzugsweise geringfügig größer als der Innenumfang. Hierdurch ergibt sich eine Übergangs- und/oder Presspassung, welche zu einer zuverlässigen und ausreichend beanspruchbaren lösbaren Verbindung führt.

In weiterer Ausgestaltung der Erfindung ist die zweite Außenfläche an einem lösbar mit der zweiten Backe verbundenen Erweiterungsteil ausgebildet, wobei die zweite Backe eine zur Anlage an dem distalen Femur nicht eingerichtete geneigte und/oder gewölbte Außenkontur aufweist, welche mittels der zweiten Außenfläche wenigstens abschnittsweise überdeckt ist. Die Messung des AP-Offsets, d.h. des Längsabstands zwischen dem distalen Femur und der proximalen Tibia, erfordert eine anforderungsgerechte Anlage der zweiten Außenfläche und der dritten Innenfläche. Zu diesem Zweck sind die besagten Flächen jeweils parallel zu der Querachse, d.h. orthogonal zu der Längsachse, längserstreckt. Eine hiervon abweichende gewölbte oder geneigte Längserstreckung erlaubt dementgegen keine anforderungsgerechte Anlage und folglich keine Messung des AP-Offsets. Die Erfinder haben erkannt, dass am Markt erhältliche Anordnungen aus Stab und Schieber, wie sie grundsätzlich zur Herstellung des erfindungsgemäßen Mehrzweckmessinstruments verwendet werden können, oftmals eine außenseitig in Bezug auf die Querachse gewölbte oder geneigte zweite Backe aufweisen. Der Außenkontur der zweiten Backe fehlt es folglich an der Eignung zur Messung des AP-Offsets. Solche am Markt erhältlichen Anordnungen können mittels des Erweiterungsteils auf einfache Weise zur Messung des AP-Offsets ertüchtigt werden. Da die zweite Außenfläche die Außenkontur der zweiten Backe wenigstens abschnittsweise überdeckt, kommt zur Messung des AP-Offsets anstelle der Außenkontur der zweiten Backe die zweite Außenfläche des Erweiterungsteils zur Anlage an dem distalen Femur.

In weiterer Ausgestaltung der Erfindung ist das Erweiterungsteil mittels einer werkzeuglos lösbaren und werkzeuglos verbindbaren zweiten Fügeverbindung mit der zweiten Backe verbunden. Hierdurch kann das Erweiterungsteil auf besonders einfache und zeitsparende Weise an der zweiten Backe angebracht und von derselben entfernt werden. Im Übrigen wird zur Vermeidung von Wiederholungen auf obige Ausführungen zur werkzeuglos lösbaren und werkzeuglos verbindbaren ersten Fügeverbindung verwiesen. Das zu der ersten Fügeverbindung Gesagte gilt mutatis mutandis auch für die zweite Fügeverbindung.

In weiterer Ausgestaltung der Erfindung weist die zweite Fügeverbindung wenigstens eine zweite Bohrung und ein zweites Bolzenelement auf, welche orthogonal zur Längs- und zur Querachse lösbar miteinander zusammengesteckt sind, wobei die zweite Bohrung in die zweite Backe eingebracht und das zweite Bolzenelement fest mit dem Erweiterungsteil verbunden ist oder umgekehrt. Die sich durch diese Ausgestaltung der zweiten Fügeverbindung ergebenden Vorteile korrespondieren mit denjenigen der entsprechenden Ausgestaltung der ersten Fügeverbindung. Zur Vermeidung von Wiederholungen wird auf die diesbezügliche Offenbarung verwiesen. Das zu der korrespondierenden Ausgestaltung der ersten Fügeverbindung Gesagte gilt mutatis mutandis auch für diese Ausgestaltung der Erfindung.

In weiterer Ausgestaltung der Erfindung sind der Stab und der Schieber jeweils aus Metall gefertigt und zur mehrfachen Verwendung eingerichtet und das Verlängerungsteil und/oder das Erweiterungsteil ist aus Kunststoff gefertigt und zur einmaligen Verwendung eingerichtet. Durch die mehrfache Verwendbarkeit der aus dem Stab und dem Schieber gebildeten Anordnung ergeben sich Kostenvorteile und eine verringerte Umweltbelastung. Dies im Vergleich zu aus dem Stand der Technik bekannten Lösungen, bei welchen das gesamte Mehrzweckmessinstrument als Wegwerfartikel zur lediglich einmaligen Verwendung eingerichtet ist.
- Fig. 1: zeigt eine schematisch stark vereinfachte Seitenansicht einer Ausführungsform eines erfindungsgemäßen Mehrzweckmessinstruments,
- Fig. 2: in schematisch stark vereinfachter Darstellung eine erste intraoperative Situation, in welcher das Mehrzweckmessinstrument nach Fig. 1 zur Messung des AP-Offsets zwischen dem distalen Femur und der proximalen Tibia verwendet wird,
- Fig. 3: in schematisch stark vereinfachter Darstellung eine zweite intraoperative Situation, in welcher das Mehrzweckmessinstrument nach den Fig. 1 und 2 zur Messung der Dicke einer femoralen Knochenresektion verwendet wird, und
- Fig. 4: in einer der Fig. 1 entsprechenden Darstellungsweise eine weitere Ausführungsform eines erfindungsgemäßen Mehrzweckmessinstruments.

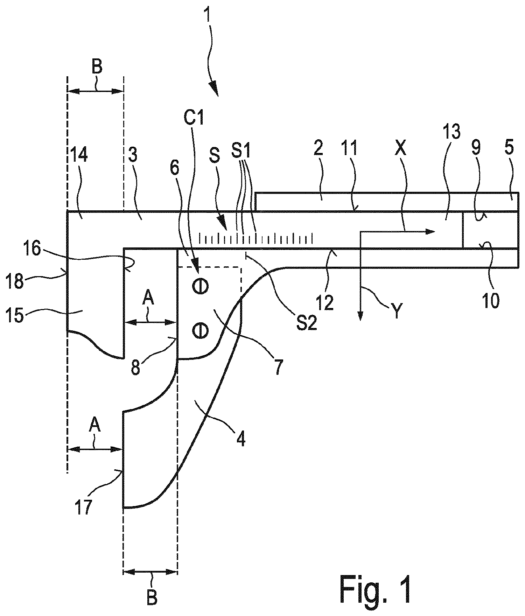

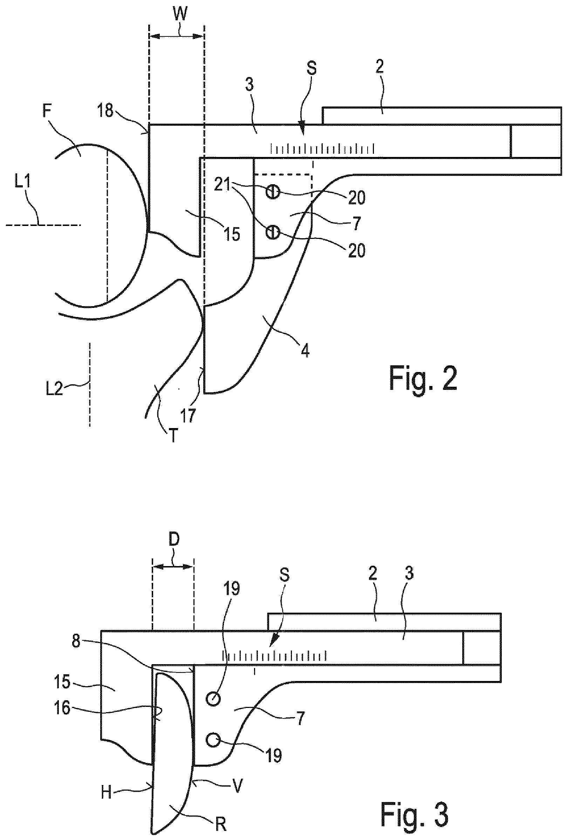

Gemäß Fig. 1 ist ein Mehrzweckmessinstrument 1 zur Verwendung bei einer Kniegelenkersatzoperation vorgesehen. Für solche Kniegelenkersatzoperationen sind unterschiedliche chirurgische Herangehensweisen etabliert. Eine Herangehensweise ist in der chirurgischen Praxis als Kinematic Alignment TKA (englisch: Total Knee Arthroplasty) bekannt. Das Mehrzweckmessinstrument 1 ist im Speziellen zur Verwendung bei einer solchen kinematically aligned TKA vorgesehen. Der besagte Operationsansatz wird nachfolgend auch als Kinematic Alignment abgekürzt. Das Kinematic Alignment umfasst mehrere intraoperative Mess- und Prüfschritte. Beispielsweise wird eingangs des operativen Eingriffs der Anterior-Posterior-Abstand zwischen dem distalen Femur F und der proximalen Tibia T unter Flexion bestimmt (siehe Fig. 2). Dieser Abstand wird auch als AP-Offset W bezeichnet. Zu Ende des operativen Eingriffs wird oftmals der AP-Offset gemessen und mit dem eingangs gemessenen Wert abgeglichen. Zudem umfasst das Kinematic Alignment eine Resektion des distalen Femurs F, wobei im Nachgang zu der Resektion die Dicke D des abgeschnittenen Knochenstücks, d.h. der femoralen Knochenresektion R, gemessen wird (siehe Fig. 3). Das Mehrzweckmessinstrument 1 dient zum einen der Messung des AP-Offsets und zum anderen der besagten Dickenmessung.

Das Mehrzweckmessinstrument 1 weist einen Stab 2, einen Schieber 3, eine zwischen dem Stab 2 und dem Schieber 3 ausgebildete Skale S und ein Verlängerungsteil 4 auf.

Der Stab 2 ist entlang einer Längsachse X zwischen einem ersten Ende 5 und einem zweiten Ende 6 längserstreckt. Einends weist der Stab 2 eine erste Backe 7 auf. Die erste Backe 7 ist im Bereich des zweiten Endes 6 angeordnet. Die erste Backe 7 ragt entlang einer Querachse Y, welche orthogonal zu der Längsachse X orientiert ist, von dem Stab 2 ab. Genauer ragt die erste Backe 7 entlang der Querachse Y von dem zweiten Ende 6 des Stabs 2 ab. Die erste Backe 7 weist eine erste Innenfläche 8 auf. Die erste Innenfläche ist parallel zu der Querachse Y gerade längserstreckt und zur Anlage an einer Vorderseite V der Knochenresektion R eingerichtet (siehe Fig. 3).

Der Schieber 3 ist entlang der Längsachse X linearbeweglich an dem Stab 2 geführt. Die Linearführung des Schiebers 3 an dem Stab 2 ist bei unterschiedlichen Ausgestaltungen unterschiedlich ausgeführt.

Bei der gezeigten Ausführungsform weist der Stab 2 eine erste Führungsbahn 9 und eine zweite Führungsbahn 10 auf. Die erste Führungsbahn 9 und die zweite Führungsbahn 10 sind jeweils parallel zu der Längsachse X zwischen dem ersten Ende 5 und dem zweiten Ende 6 längserstreckt und liegen einander entlang der Querachse Y gegenüber. Der Schieber 3 weist eine erste Führungsfläche 11 und eine zweite Führungsfläche 12 auf. Die beiden Führungsflächen 11, 12 sind parallel zu der Längsachse X längserstreckt und in Bezug auf die Querachse Y einander gegenüberliegend angeordnet. Die erste Führungsfläche 11 ist entlang der ersten Führungsbahn 9 gleitbeweglich. Die zweite Führungsfläche 12 ist entlang der zweiten Führungsbahn 10 gleitbeweglich. Gleichzeitig ist der Schieber 3 in Bezug auf die Querrichtung Y formschlüssig zwischen den beiden Führungsbahnen 9, 10 gehalten. Die detaillierte Funktion und konstruktive Umsetzung der Linearführung des Schiebers ist in Bezug auf die vorliegende Erfindung nicht wesentlich. Auf weitere diesbezügliche Erläuterungen kann deshalb verzichtet werden.

Der Schieber 3 ist entlang der Längsachse X zwischen seinem ersten Ende 13 und seinem zweiten Ende 14 längserstreckt und weist eine von dem zweiten Ende 14 parallel zu der Querachse Y abragende zweite Backe 15 auf. Die zweite Backe 15 weist eine zweite Innenfläche 16 auf, welche parallel zu der Querachse Y gerade längserstreckt und entgegengesetzt zu der ersten Innenfläche 8 orientiert ist. Die zweite Innenfläche 16 ist zur Anlage an einer Rückseite H der Knochenresektion R eingerichtet (siehe Fig. 3).

Die erste Backe 7 und die zweite Backe 15 ragen jeweils parallel zu der Querachse Y in eine gemeinsame Richtung von dem Stab 2 bzw. dem Schieber 3 ab. In Bezug auf die Zeichenebene der Fig. 1 ragen beide Backen 7, 8 nach unten. Die erste Innenfläche 8 und die zweite Innenfläche 16 sind zueinander parallel längserstreckt und zueinander entgegengesetzt orientiert. Die erste Innenfläche 8 weist - in Bezug auf die Zeichenebene der Fig. 1 - nach links, die zweite Innenfläche 16 weist nach rechts.

Die Skale S ist zwischen dem Stab 2 und dem Schieber 3 ausgebildet und zur Anzeige der Dicke D der Knochenresektion R eingerichtet (siehe Fig. 3). Zu diesem Zweck ist die Skale S der ersten Innenfläche 8 und der zweiten Innenfläche 16 zugeordnet und zeigt deren Abstand A in Bezug auf die Längsachse X an. Unterschiedliche Möglichkeiten zur Gestaltung der Skale S sind dem einschlägigen Fachmann prinzipiell bekannt.

Bei der gezeigten Ausführungsform weist die Skale S eine Mehrzahl von entlang der Längsachse X aufeinanderfolgenden Teilstrichen S1 auf. Zudem weist die Skale S eine an dem Stab 2 angeordnete Ablesemarke S2 auf. Der sich jeweils ergebende und/oder gemessene Abstand A kann an der Ablesemarke S2, genauer: an dem unmittelbar oberhalb der Ablesemarke S2 befindlichen Teilstrich, abgelesen werden.

Das Verlängerungsteil 4 ist lösbar mit der ersten Backe 7 verbunden und weist eine dritte Innenfläche 17 auf. Die dritte Innenfläche 17 ist parallel zu der Querachse Y gerade längserstreckt und zur Anlage an der proximalen Tibia T eingerichtet (siehe Fig. 2). Das Verlängerungsteil 4 ragt in Bezug auf die Querachse Y weiter von dem Stab 2 ab als die erste Backe 7. Sowohl die erste Backe 7 als auch das Verlängerungsteil 4 ragen auf ein- und dieselbe Seite des Stabs 2 ab. Vorliegend und in Bezug auf die Zeichenebene der Fig. 1 ragen und/oder weisen die erste Backe 7 und das Verlängerungsteil 4 jeweils nach unten. Die dritte Innenfläche 17 ist folglich entlang der Querachse Y weiter von der Längsachse X beabstandet als die erste Innenfläche 8.

Die zweite Backe 15 weist eine zweite Außenfläche 18 auf, die parallel zu der zweiten Innenfläche 16 gerade längserstreckt und entgegengesetzt zu derselben orientiert ist. Die zweite Außenfläche 18 ist zur Anlage an dem distalen Femur F eingerichtet (vgl. Fig. 2).

Die erste Innenfläche 8 und die dritte Innenfläche 17 sind entlang der Längsachse X um einen Abstand B voneinander beabstandet. Die zweite Innenfläche 16 und die zweite Außenfläche 18 sind hierzu identisch und demnach ebenfalls um den Abstand B voneinander beabstandet. Infolge dieser in Bezug auf die Längsachse X paarweise identischen Beabstandung zwischen der ersten Innenfläche 8 und der dritten Innenfläche 17 einerseits und der zweiten Innenfläche 16 und der zweiten Außenfläche 18 andererseits ist die Skale S zusätzlich zur Anzeige des Längsabstands zwischen der dritten Innenfläche 17 und der zweiten Außenfläche 18 eingerichtet. Mit anderen Worten ausgedrückt, sind die erste Innenfläche 8 und die zweite Innenfläche 16 einerseits und die dritte Innenfläche 17 und die zweite Außenfläche 18 andererseits stets gleichermaßen um den (veränderlichen) Abstand A voneinander beabstandet.

Die Skale S ist folglich gleichermaßen zur Anzeige des AP-Offsets W und der Dicke D eingerichtet.

Wie anhand der Fig. 2 und 3 gezeigt ist, erfolgt die Messung des AP-Offsets W zum einen und die Messung der Dicke D der Knochenresektion R zum anderen vorliegend in unterschiedlichen Konfigurationen des Mehrzweckmessinstruments 1. In diesem Zusammenhang kann auch von einer ersten Konfiguration (Fig. 2) und einer zweiten Konfiguration gesprochen werden (Fig. 3).

In der ersten Konfiguration ist das Verlängerungsteil 4 lösbar mit der ersten Backe 7 verbunden. Zur Messung des AP-Offsets W wird das Mehrzweckmessinstrument 1 manuell und mit der dritten Innenfläche 17 voran an die proximale Tibia T angelegt. Hierbei wird die Längsachse X des Mehrzweckmessinstruments 1 vorzugsweise parallel zu einer Längsachse L1 des Femurs ausgerichtet. Die Messung des AP-Offsets W erfolgt üblicherweise unter Flexion, so dass eine Längsachse L2 der Tibia in etwa orthogonal zu der Längsachse L1 des Femurs und damit auch zu der Längsachse X des Mehrzweckmessinstruments 1 ausgerichtet ist. Nach Anlage der dritten Innenfläche 17 an der proximalen Tibia T wird der Schieber 3 relativ zu dem Stab 2 so weit in Richtung des distalen Femurs F verlagert, bis die zweite Außenfläche 18 zur Anlage gelangt. Der AP-Offset W kann dann auf die vorbeschriebene Weise an der Skale S abgelesen werden.

Im weiteren Verlauf des Kinematic Alignment wird der distale Femur üblicherweise reseziert. Die Resektion erfolgt üblicherweise orthogonal zur Längsachse L1 des Femurs, wie dies in Fig. 2 mittels der dort strichlierten Linie angedeutet ist. Die sich dann ergebende Knochenresektion R ist in Fig. 3 gezeigt. Dabei erfolgt die Messung der Dicke D der Knochenresektion R in der zweiten Konfiguration des Mehrzweckmessinstruments 1.

In der zweiten Konfiguration ist das Verlängerungsteil 4 von der ersten Backe 7 gelöst und/oder entfernt. Zur Dickenmessung wird die Knochenresektion R zwischen die erste Backe 7 und die zweite Backe 15 geführt und der Schieber 3 wird relativ zu dem Stab 2 in Richtung des ersten Endes 5 längsverschoben. Dies, bis die erste Innenfläche 8 an der Vorderseite V und die zweite Innenfläche 16 an der Rückseite H der Knochenresektion R zur Anlage gelangen. Der (maximale) Längsabstand zwischen der Vorderseite V und der Rückseite H entspricht der Dicke D, welche auf die vorbeschriebene Weise an der Skale S abgelesen werden kann.

Zur erneuten Messung des AP-Offsets an dann implantierten künstlichen Gelenkkomponenten kann das Verlängerungsteil 4 ausgehend von der zweiten Konfiguration erneut mit der ersten Backe 7 verbunden werden.

Wie weiter anhand der Fig. 2 und 3 erkennbar ist, ist die erste Innenfläche 8 ungeeignet zur Messung des AP-Offsets. Vielmehr ist ein gewisser Querabstand der instrumentenseitigen Anlagefläche von der Längsachse X erforderlich, um eine gleichzeitige Anlage an dem distalen Femur F einerseits und der proximalen Tibia T andererseits zu ermöglichen. Zu diesem Zweck ragt das Verlängerungsteil 4 weiter von der Längsachse X ab als die erste Backe 7. Umgekehrt ist eine Dickenmessung zwischen der dritten Innenfläche 17 und der zweiten Innenfläche 16 nicht möglich (Fig. 2). Denn die besagten Innenflächen liegen einander nicht unmittelbar gegenüber. Vielmehr ist die dritte Innenfläche 17 in Bezug auf die Querachse Y nach unten versetzt zu der zweiten Innenfläche 16 angeordnet und umgekehrt. Hierzu im Unterschied liegen die erste Innenfläche 8 und die zweite Innenfläche 16 einander unmittelbar gegenüber.

Dies erlaubt eine anforderungsgerechte Dickenmessung (siehe Fig. 3). Zu diesem Zweck ist das Verlängerungsteil 4 auf einfache Weise entfernbar.

Um ein möglichst einfaches Anbringen und Entfernen an der ersten Backe 7 zu ermöglichen, ist das Verlängerungsteil 4 mittels einer werkzeuglosen ersten Fügeverbindung C1 mit der ersten Backe 7 verbunden. Werkzeuglos meint, dass die erste Fügeverbindung C1 ohne die Verwendung eines Werkzeugs lösbar und verbindbar ist.

Die erste Fügeverbindung C1 ist bei unterschiedlichen Ausgestaltungen unterschiedlich ausgeführt und kann beispielsweise als Rast-, Steck- oder Klemmverbindung gestaltet sein.

Bei der gezeigten Ausführungsform ist die erste Fügeverbindung C1 eine Steckverbindung. Die Steckverbindung ist zwischen hierfür geeigneten Abschnitten und/oder Bauteilen der ersten Backe 7 einerseits und des Verlängerungsteils 4 andererseits ausgebildet.

Bei der gezeigten Ausführungsform weist die erste Fügeverbindung C1 zwei erste Bohrungen 19 und zwei erste Bolzenelemente 20 auf. Die ersten Bohrungen 19 und die ersten Bolzenelemente 20 sind orthogonal zur Längsachse X und orthogonal zur Querachse Y miteinander zusammengesteckt. Vorliegend sind die ersten Bohrungen 19 in die erste Backe 7 eingebracht (siehe Fig. 3). Die ersten Bolzenelemente 20 sind fest mit dem Verbindungsteil 4 verbunden und bei der gezeigten Ausführungsform einstückig an demselben ausgebildet. Die ersten Bohrungen 19 sind jeweils als Durchgangsbohrung ausgeführt und zwischen einander gegenüberliegenden Außenseiten der ersten Backe 7 durchgängig erstreckt. Die ersten Bolzenelemente 20 ragen jeweils seitlich und orthogonal zur Längs- und zur Querachse von dem Verlängerungsteil 4 auf. Die ersten Bolzen 20, genauer: deren Außenumfang und/oder Durchmesser, sind maßlich auf die ersten Bohrungen 19 abgestimmt und umgekehrt. Im Speziellen ist der Durchmesser der ersten Bolzenelemente 20 geringfügig größer als der Innendurchmesser der ersten Bohrungen 19. Dies gewährleistet einen festen, aber dennoch lösbaren Sitz des Verlängerungsteils 4.

Es versteht sich, dass unterschiedliche Ausgestaltungen unterschiedliche Anzahlen von Bohrungen und Bolzenelementen aufweisen können. Bei einer in den Figuren nicht gezeigten Ausführungsform sind folglich lediglich eine einzige Bohrung und ein einziges Bolzenelement vorhanden.

Bei der gezeigten Ausführungsform weisen die ersten Bolzenelemente 20 jeweils einen stirnendseitig eingebrachten Schlitz 21 auf. Der Schlitz 21 unterstützt eine elastische Federbewegung der ersten Bolzenelemente 20 in radialer Richtung. Diese elastische Federbewegung sorgt für eine ausreichende Nachgiebigkeit beim Einstecken der ersten Bolzenelemente 20 in die ersten Bohrungen 19.

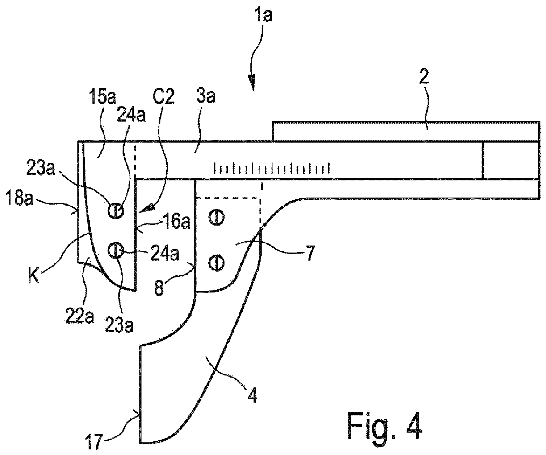

Anhand Fig. 4 ist eine weitere Ausführungsform eines erfindungsgemäßen Mehrzweckmessinstruments 1a gezeigt. Das Mehrzweckmessinstrument 1a nach Fig. 4 weist eine weitestgehende Übereinstimmung mit dem Mehrzweckmessinstrument 1 nach den Fig. 1 bis 3 auf. Nachfolgend wird lediglich auf wesentliche Unterschiede des Mehrzweckmessinstruments 1a nach Fig. 4 gegenüber dem Mehrzweckmessinstrument 1 eingegangen. Identische Bauteile und/oder Abschnitte sind mit identischen Bezugszeichen versehen und werden nicht gesondert erläutert. Hinsichtlich ihrer Gestaltung und/oder Funktion unterschiedliche Bauteile und/oder Abschnitte sind mit identischen Bezugszeichenziffern unter Hinzufügung eines Kleinbuchstabens gekennzeichnet.

Das Mehrzweckmessinstrument 1a unterscheidet sich durch einen unterschiedlich gestalteten Schieber 3a von dem Mehrzweckmessinstrument 1. Der Schieber 3a weist eine zweite Backe 15a auf. Die zweite Backe 15a weist eine zur Anlage an dem distalen Femur F nicht eingerichtete Außenkontur K auf. Die Außenkontur K ist insoweit geneigt und/oder gewölbt. Die besagte Neigung und/oder Wölbung verhindert eine anforderungsgerechte Anlage an dem distalen Femur (siehe Fig. 2). Um dennoch eine Messung des AP-Offsets W zu ermöglichen, weist das Mehrzweckmessinstrument 1a ein lösbar mit der zweiten Backe 15a verbundenes Erweiterungsteil 22a auf, an welchem die zweite Außenfläche 18a ausgebildet ist. Hinsichtlich der übrigen Funktion und Gestaltung der zweiten Außenfläche 18a wird auf das zu der Ausführungsform nach den Fig. 1 bis 3 Gesagte verwiesen.

Im Unterschied zu der Außenkontur K ist die zweite Außenfläche 18a parallel zu der Querachse Y gerade längserstreckt. Dies ermöglicht die anforderungsgerechte Anlage an dem distalen Femur F. Die Außenkontur K wird von der zweiten Außenfläche 18a wenigstens abschnittsweise überdeckt. Mit anderen Worten ausgedrückt, überragt das Erweiterungsteil 22a die zweite Backe 15a in Bezug auf die Längsachse X. Das Erweiterungsteil 22a bildet insoweit ein vorderes Stirnende des Mehrzweckmessinstruments 1a.

Das Erweiterungsteil 22a ist mittels einer zweiten Fügeverbindung C2 lösbar mit der zweiten Backe 15a verbunden. Die zweite Fügeverbindung C2 ist bei unterschiedlichen Ausgestaltungen unterschiedlich ausgeführt. Denkbar ist wiederum eine Rast-, Klemm- oder Steckverbindung.

Bei der gezeigten Ausführungsform ist die zweite Fügeverbindung C2 analog zu der ersten Fügeverbindung C1 gestaltet und weist dementsprechend zwei zweite Bohrungen 23a und zwei zweite Bolzenelemente 24a auf. Im Übrigen wird zur Vermeidung von Wiederholungen auf das zu der ersten Fügeverbindung Gesagte verwiesen.

Bei den gezeigten Ausführungsformen sind der Stab 2 und der jeweilige Schieber 3, 3a jeweils aus Metall gefertigt. Hierzu im Unterschied sind das Verlängerungsteil 4 und das Erweiterungsteil 22a jeweils aus Kunststoff gefertigt. Durch die Fertigung aus Metall sind die Anordnungen aus Stab 2 und Schieber 3, 3a jeweils zur mehrfachen Verwendung eingerichtet. Das Verlängerungsteil 4 und das Erweiterungsteil 22a sind dementgegen zur einmaligen Verwendung vorgesehen.

## Patentansprüche

1. Mehrzweckmessinstrument (1, 1a) zur Verwendung bei einer Kniegelenkersatzoperation, aufweisend
einen Stab (2), welcher entlang einer Längsachse (X) längserstreckt ist und einends eine entlang einer Querachse (Y) abragende erste Backe (7) aufweist, wobei die erste Backe (7) eine parallel zu der Querachse (Y) erstreckte erste Innenfläche (8) aufweist, welche zur Anlage an einer Vorderseite (V) einer Knochenresektion (R) eingerichtet ist,
einen Schieber (3, 3a), welcher entlang der Längsachse (X) linearbeweglich an dem Stab (2) geführt ist und einends eine entlang der Querachse (Y) abragende zweite Backe (15, 15a) aufweist, wobei die zweite Backe (15, 15a) eine parallel zu der Querachse (Y) erstreckte zweite Innenfläche (16, 16a) aufweist, welche entgegengesetzt zu der ersten Innenfläche (8) orientiert und zur Anlage an einer Rückseite (H) der Knochenresektion (R) eingerichtet ist,
und aufweisend eine Skale (S), welche zwischen dem Stab (2) und dem Schieber (3, 3a) ausgebildet und wenigstens zur Anzeige einer entlang der Längsachse (X) erstreckten Dicke (D) der Knochenresektion (R) eingerichtet ist,
**dadurch gekennzeichnet,**
**dass** ein Verlängerungsteil (4) lösbar mit der ersten Backe (7) verbunden ist, wobei das Verlängerungsteil (4) eine zur Anlage an einer proximalen Tibia (T) eingerichtete dritte Innenfläche (17) aufweist, die parallel zu der Querachse (Y) erstreckt ist und weiter von dem Stab (2) abragt als die erste Innenfläche (8),
und **dass** die zweite Backe (15, 15a) eine parallel zu der Querachse (Y) erstreckte zweite Außenfläche (18, 18a) aufweist, welche entgegengesetzt zu der zweiten Innenfläche (16, 16a) orientiert und zur Anlage an einem distalen Femur (F) eingerichtet ist,
wobei die dritte Innenfläche (17) und die erste Innenfläche (8) zum einen und zum anderen die zweite Innenfläche (16, 16a) und die zweite Außenfläche (18, 18a) jeweils entlang der Längsachse (X) um einen identischen Abstand (B) voneinander beabstandet sind,
wodurch die Skale (S) zusätzlich zur Anzeige eines Längsabstands (W) zwischen dem distalen Femur (F) und der proximalen Tibia (T) eingerichtet ist.

2. Mehrzweckmessinstrument (1, 1a) nach Anspruch 1, **dadurch gekennzeichnet, dass** das Verlängerungsteil (4) mittels einer werkzeuglos lösbaren und werkzeuglos verbindbaren ersten Fügeverbindung (C1) mit der ersten Backe (7) verbunden ist.

3. Mehrzweckmessinstrument (1, 1a) nach Anspruch 2, **dadurch gekennzeichnet, dass** die erste Fügeverbindung (C1) wenigstens eine erste Bohrung (19) und ein erstes Bolzenelement (20) aufweist, welche orthogonal zur Längs- und zur Querachse lösbar miteinander zusammengesteckt sind, wobei die erste Bohrung (19) in die erste Backe (7) eingebracht ist und das erste Bolzenelement (20) fest mit dem Verlängerungsteil (4) verbunden ist oder umgekehrt.

4. Mehrzweckmessinstrument (1a) nach einem der vorhergehenden Ansprüche, **dadurch gekennzeichnet, dass** die zweite Außenfläche (18a) an einem lösbar mit der zweiten Backe (15a) verbundenen Erweiterungsteil (22a) ausgebildet ist, wobei die zweite Backe (15a) eine zur Anlage an dem distalen Femur (F) nicht eingerichtete geneigte und/oder gewölbte Außenkontur (K) aufweist, welche mittels der zweiten Außenfläche (18a) wenigstens abschnittsweise überdeckt ist.

5. Mehrzweckmessinstrument (1a) nach Anspruch 4, **dadurch gekennzeichnet, dass** das Erweiterungsteil (22a) mittels einer werkzeuglos lösbaren und werkzeuglos verbindbaren zweiten Fügeverbindung (C2) mit der zweiten Backe (15a) verbunden ist.

6. Mehrzweckmessinstrument (1a) nach Anspruch 5, **dadurch gekennzeichnet, dass** die zweite Fügeverbindung (C2) wenigstens eine zweite Bohrung (23a) und ein zweites Bolzenelement (24a) aufweist, welche orthogonal zur Längs- und zur Querachse lösbar miteinander zusammengesteckt sind, wobei die zweite Bohrung (23a) in die zweite Backe (15a) eingebracht ist und das zweite Bolzenelement (24a) fest mit dem Erweiterungsteil (22a) verbunden ist oder umgekehrt.

7. Mehrzweckmessinstrument (1, 1a) nach einem der vorhergehenden Ansprüche, **dadurch gekennzeichnet, dass** der Stab (2) und der Schieber (3, 3a) jeweils aus Metall gefertigt und zur mehrfachen Verwendung eingerichtet sind, und dass das Verlängerungsteil (4) und/oder das Erweiterungsteil (22a) aus Kunststoff gefertigt und zur einmaligen Verwendung eingerichtet ist.

## Claims

1. Multipurpose measurement instrument (1, 1a) for use in a knee-joint replacement operation, having
a rod (2) which extends along a longitudinal axis (X) and at one end has a first jaw (7) protruding along a transverse axis (Y), wherein the first jaw (7) has a first inner surface (8) which extends parallel to the transverse axis (Y) and which is configured to bear on a front face (V) of a bone resection (R),
a slide (3, 3a) which is guided linearly movably on the rod (2) along the longitudinal axis (X) and at one end has a second jaw (15, 15a) protruding along the transverse axis (Y), wherein the second jaw (15, 15a) has a second inner surface (16, 16a) which extends parallel to the transverse axis (Y) and which is oriented counter to the first inner surface (8) and is configured to bear on a rear face (H) of the bone resection (R),
and having a scale (S) which is formed between the rod (2) and the slide (3, 3a) and is configured at least to indicate a thickness (D) of the bone resection (R) along the longitudinal axis (X),
**characterized in that**
a continuation part (4) is connected releasably to the first jaw (7), wherein the continuation part (4) has a third inner surface (17) which is configured to bear on a proximal tibia (T) and which extends parallel to the transverse axis (Y) and protrudes further from the rod (2) than does the first inner surface (8),
and the second jaw (15, 15a) has a second outer surface (18, 18a) which extends parallel to the transverse axis (Y) and which is oriented counter to the second inner surface (16, 16a) and is configured to bear on a distal femur (F),
wherein on the one hand the third inner surface (17) and the first inner surface (8) and on the other hand the second inner surface (16, 16a) and the second outer surface (18, 18a) are in each case spaced apart from each other along the longitudinal axis (X) by an identical distance (B),
as a result of which the scale (S) is additionally configured to indicate a longitudinal distance (W) between the distal femur (F) and the proximal tibia (T).

2. Multipurpose measurement instrument (1, 1a) according to Claim 1, **characterized in that** the continuation part (4) is connected to the first jaw (7) by means of a first joining connection (C1) that is releasable without tools and connectable without tools.

3. Multipurpose measurement instrument (1, 1a) according to Claim 2, **characterized in that** the first joining connection (C1) has at least a first bore (19) and a first pin element (20) which are releasably plugged together orthogonally with respect to the longitudinal axis and the transverse axis, wherein the first bore (19) is introduced into the first jaw (7), and the first pin element (20) is rigidly connected to the continuation part (4), or vice versa.

4. Multipurpose measurement instrument (1a) according to one of the preceding claims, **characterized in that** the second outer surface (18) is formed on an extension part (22a) connected releasably to the second jaw (15a), wherein the second jaw (15a) has an inclined and/or curved outer contour (K) which is not configured to bear on the distal femur (F) and which is covered at least in part by means of the second outer surface (18a).

5. Multipurpose measurement instrument (1a) according to Claim 4, **characterized in that** the extension part (22a) is connected to the second jaw (15a) by means of a second joining connection (C2) that is releasable without tools and connectable without tools.

6. Multipurpose measurement instrument (1a) according to Claim 5, **characterized in that** the second joining connection (C2) has at least a second bore (23a) and a second pin element (24a) which are releasably plugged together orthogonally with respect to the longitudinal axis and the transverse axis, wherein the second bore (23a) is introduced into the second jaw (15a), and the second pin element (24a) is rigidly connected to the extension part (22a), or vice versa.

7. Multipurpose measurement instrument (1, 1a) according to one of the preceding claims, **characterized in that** the rod (2) and the slide (3, 3a) are each made of metal and are configured for repeated use, and **in that** the continuation part (4) and/or the extension part (22a) are/is made of plastic and configured to be used just once.

## Revendications

1. Instrument de mesure polyvalent (1, 1a) destiné à être utilisé en chirurgie de remplacement du genou, comportant
une tige (2), qui s'étend longitudinalement le long d'un axe longitudinal (X) et comporte sur une extrémité une première mâchoire (7) faisant saillie le long d'un axe transversal (Y), la première mâchoire (7) comportant une première surface intérieure (8) s'étendant parallèlement à l'axe transversal (Y), qui est mise au point pour venir en appui sur une face avant (V) d'une résection osseuse (R),
un coulisseau (3, 3a), qui est guidé de manière mobile linéairement sur la tige (2) le long de l'axe longitudinal (X) et comporte sur une extrémité une deuxième mâchoire (15, 15a) faisant saillie le long de l'axe transversal (Y), la deuxième mâchoire (15, 15a) comportant une deuxième surface intérieure (16, 16a) s'étendant parallèlement à l'axe transversal (Y), laquelle est orientée à l'opposé de la première surface intérieure (8) et est mise au point pour venir en appui sur une face arrière (H) de la résection osseuse (R), et comportant une échelle (S), qui est formée entre la tige (2) et le coulisseau (3, 3a) et est mise au point au moins pour indiquer une épaisseur (D) de la résection osseuse (R) s'étendant le long de l'axe longitudinal (X), **caractérisé en ce**
**qu'**une partie de prolongement (4) est reliée de manière amovible à la première mâchoire (7), la partie de prolongement (4) comportant une troisième surface intérieure (17) mise au point pour venir en appui sur un tibia proximal (T), laquelle s'étend parallèlement à l'axe transversal (Y) et fait davantage saillie de la tige (2) que la première surface intérieure (8),
et **que** la deuxième mâchoire (15, 15a) comporte une deuxième surface extérieure (18, 18a) s'étendant parallèlement à l'axe transversal (Y), laquelle est orientée à l'opposé de la deuxième surface intérieure (16, 16a) et est mise au point pour venir en appui sur un fémur distal (F),
la troisième surface intérieure (17) et la première surface intérieure (8) d'une part et la deuxième surface intérieure (16, 16a) et la deuxième surface extérieure (18, 18a) d'autre part étant espacées l'une de l'autre d'une distance (B) identique respectivement le long de l'axe longitudinal (X),
l'échelle (S) étant ainsi mise au point en supplément pour afficher une distance longitudinale (W) entre le fémur distal (F) et le tibia proximal (T).

2. Instrument de mesure polyvalent (1, 1a) selon la revendication 1, **caractérisé en ce que** la partie de prolongement (4) est reliée à la première mâchoire (7) au moyen d'un premier raccord d'assemblage (C1) amovible sans outil et pouvant être relié sans outil.

3. Instrument de mesure polyvalent (1, 1a) selon la revendication 2, **caractérisé en ce que** le premier raccord d'assemblage (C1) comporte au moins un premier alésage (19) et un premier élément de boulon (20), qui sont assemblés l'un à l'autre par emboîtement de manière amovible orthogonalement par rapport à l'axe longitudinal et à l'axe transversal, le premier alésage (19) étant introduit dans la première mâchoire (7) et le premier élément de boulon (20) étant relié de manière solidaire à la partie de prolongement (4) ou inversement.

4. Instrument de mesure polyvalent (1a) selon l'une des revendications précédentes, **caractérisé en ce que** la deuxième surface extérieure (18a) est formée sur une partie d'élargissement (22a) reliée de manière amovible à la deuxième mâchoire (15a), la deuxième mâchoire (15a) comportant un contour extérieur (K) incliné et/ou incurvé qui n'est pas mis au point pour venir en appui sur le fémur distal (F), lequel est au moins par endroits recouvert au moyen de la deuxième surface extérieure (18a).

5. Instrument de mesure polyvalent (1a) selon la revendication 4, **caractérisé en ce que** la partie d'élargissement (22a) est reliée à la deuxième mâchoire (15a) au moyen d'un deuxième raccord d'assemblage (C2) amovible sans outil et pouvant être relié sans outil.

6. Instrument de mesure polyvalent (1a) selon la revendication 5, **caractérisé en ce que** le deuxième raccord d'assemblage (C2) comporte au moins un deuxième alésage (23a) et un deuxième élément de boulon (24a), qui sont assemblés l'un à l'autre par emboîtement de manière amovible orthogonalement par rapport à l'axe longitudinal et à l'axe transversal, le deuxième alésage (23a) étant introduit dans la deuxième mâchoire (15a) et le deuxième élément de boulon (24a) étant relié de manière solidaire à la partie d'élargissement (22a) ou inversement.

7. Instrument de mesure polyvalent (1, 1a) selon l'une des revendications précédentes, **caractérisé en ce que** la tige (2) et le coulisseau (3, 3a) sont chacun fabriqués en métal et sont mis au point pour un usage multiple, et que la partie de prolongement (4) et/ou la partie d'élargissement (22a) sont fabriquées en matière plastique et sont mises au point pour un usage unique.
